## Europäisches Patentamt
## European Patent Office
## Office européen des brevets

(19)

(11) Veröffentlichungsnummer: **0 022 493**
**B1**

(12) **EUROPÄISCHE PATENTSCHRIFT**

(45) Veröffentlichungstag der Patentschrift:
**14.03.84**

(51) Int. Cl.³: **G 01 N 11/08**, G 01 N 33/22,
F 23 N 5/18

(21) Anmeldenummer: **80103586.6**

(22) Anmeldetag: **25.06.80**

(54) **Verfahren und Vorrichtung zur verbrennungslosen Messung und/oder Regelung der Wärmemengenzufuhr zu Gasverbrauchseinrichtungen.**

(30) Priorität: **17.07.79 DE 2928739**

(43) Veröffentlichungstag der Anmeldung:
**21.01.81 Patentblatt 81/3**

(45) Bekanntmachung des Hinweises auf die Patenterteilung:
**14.03.84 Patentblatt 84/11**

(84) Benannte Vertragsstaaten:
**FR GB IT**

(56) Entgegenhaltungen:
**DE - A - 1 476 788**
**DE - A - 2 011 717**
**DE - A - 2 745 459**
**FR - A - 1 129 398**
**US - A - 3 334 513**

**NASA TECH BRIEF, B75-10004, März 1975,**

(73) Patentinhaber: **Ruhrgas Aktiengesellschaft,
Huttropstrasse 60 Postfach 10 32 52,
D-4300 Essen 1 (DE)**

(72) Erfinder: **Sommers, Hans, Dipl.-Ing.,
Friedrich-List-Strasse 9, D-4300 Essen 1 (DE)**
Erfinder: **Korsmeier, Wilhelm, Spiekeroogstrasse 5,
D-4350 Recklinghausen (DE)**
Erfinder: **Vissel, Friedrich Dr.rer.nat, Dipl.Phys.,
Belsingerweg 77, D-4300 Recklinghausen (DE)**

BUNDESDRUCKEREI BERLIN

## Verfahren und Vorrichtung zur verbrennungslosen Messung und/oder Regelung der Wärmemengenzufuhr zu Gasverbrauchseinrichtungen

Die Erfindung betrifft Verfahren zur verbrennungslosen Messung und/oder Regelung der Wärmemengenzufuhr zu Gasverbrauchseinrichtungen, insbesondere Erdgasverbrauchseinrichtungen in mehreren Verfahrensvarianten sowie Vorrichtungen zur Durchführung der verschiedenen Verfahrensvarianten.

Zur Messung des Brennwertes oder anderer Eigenschaften von Brenngasen, wie Heizwert oder Wobbe-Index, war es bisher üblich, einen Teilstrom des Brenngases in einem geeigneten Gerät (Kalorimeter) mit freier Flamme oder an einem Katalysator zu verbrennen und die dabei entstehende Wärme zu messen. Der von diesen Geräten gelieferte Meßwert kann zur Anzeige der erwähnten Gaseigenschaften oder als Signal für Regelvorgänge verwendet werden, zum Beispiel um den Brennwert oder den Wobbe-Index eines Gasstromes durch Zuführung anderer Gase konstant zu halten oder den Gasmengenstrom in zweckmäßiger Weise zu verändern.

Die Notwendigkeit, einen gemessenen Teilstrom des Gases zu verbrennen, um den Brennwert oder den Wobbe-Index zu messen, erfordert erfahrungsgemäß eine häufige Wartung der Geräte, weil sich die Flamme durch Ansätze von Verbrennungsrückständen verändern kann beziehungsweise weil Verbrennungskatalysatoren allmählich an Wirksamkeit verlieren. Die erforderliche Genauigkeit von Messungen, die zum Beispiel für Abrechnungszwecke dienen, kann nur erreicht werden, wenn diese Geräte unter definierten und kontrollierten Bedingungen — vorzugsweise in einem klimatisierten Raum — betrieben werden, was ebenfalls einen großen Aufwand erfordert.

Es sind auch Methoden bekannt, einen Gasstrom ohne Verbrennung kontinuierlich zu analysieren, z. B. durch Absorption verschiedener Gasbestandteile in geeigneten Lösungsmitteln, durch Gaschromatographie oder durch Messung der Absorption ultraroter Strahlung. Aus den kontinuierlich ermittelten Anteilen verschiedener Gase im Gasstrom kann dann zum Beispiel mittels eines elektronischen Rechenwerkes der Brennwert, der Heizwert oder der Wobbe-Index des untersuchten Gases kontinuierlich angezeigt oder als Regelimpuls verwendet werden. Diese Methoden erfordern jedoch einen großen Aufwand an Meß- und Steuereinrichtungen.

Verfahren zur Wärmemengenmessung werden in der Praxis heute schon zu zahlreichen Zwecken verwendet. In jüngster Zeit ist das Interesse beziehungsweise der Bedarf an solchen Messungen aus verschiedenen Gründen erheblich gestiegen: Bei industriellen Wärmeprozessen ist es häufig notwendig, einem Ofen eine genau definierte Wärmemenge pro Zeiteinheit zuzuführen, um optimale Ergebnisse zu erzielen. In anderern Fällen wird angestrebt, den Brennstoffverbrauch zu optimieren, das heißt nur die tatsächlich benötigte Wärmemenge zuzuführen, auch wenn eine größere Wärmezufuhr den Prozeß beziehungsweise das Produkt nicht negativ beeinflußt. Für Abrechnungszwecke wird neuerdings ebenfalls die Abrechnung auf der Basis der gelieferten Wärmemenge derjenigen auf Volumen-Basis vorgezogen.

Für die vorstehend beispielhaft genannten Anwendungsfälle für Wärmemengenmessungen ist ein unfangreicher Bedarf unter anderem dadurch entstanden, daß Brenngase, insbesondere Erdgase unterschiedlicher Herkunft, verteilt werden, deren Zusammensetzung und Eigenschaften sich mehr oder weniger stark voneinander unterscheiden. Nachdem Verfahren und Vorrichtungen entwickelt und erprobt worden sind, um solche Gase trotz dieser Unterschiede in Gasverbrauchseinrichtungen einzusetzen, ohne daß bei Änderung der Gasqualität Änderungen an den Verbrauchseinrichtungen vorgenommen werden müssen, gewinnt die Wärmemengenmessung sowohl beim industriellen Gaseinsatz als auch für Abrechnungszwecke zunehmende Bedeutung.

Die bekannten Verfahren sind aber für die neuen Zwecke aus technischen oder Kostengründen vielfach nicht anwendbar.

Der Erfindung liegt die Aufgabe zugrunde, die Messung von Brenngas-Eigenschaften wie Brennwert, Heizwert oder Wobbe-Index ohne Verbrennung von Gas und auf einfachere Weise als mit den bisher bekannten verbrennungslosen Methoden durchzuführen. Eine weitere Aufgabe, die durch die Erfindung gelöst wird, besteht darin, daß bei Änderung der Gasqualität der oder den nachgeschalteten Gasverbrauchseinrichtung(en) eine vorgegebene Wärmemenge durch Änderung des Gasvolumenstromes weiterhin richtig zugeführt wird.

Die nachstehend beschriebene Erfindung basiert auf der überraschenden Erkenntnis, daß der Wobbe-Index von Erdgasen verschiedener Herkunft und Zusammensetzung sowie von anderen Brenngasen mit großer Genauigkeit eine Funktion der Viskosität dieser Gase ist.

Der obere Norm-Wobbe-Index $W_{o,n}$ und der obere Betrieb-Wobbe-Index $W_o$ sind bekanntlich definiert durch die Ausdrücke

$$W_{o,n} = \frac{H_{o,n}}{\sqrt{d_v}} \tag{1}$$

$$W_o = \frac{H_o}{\sqrt{d_v}} \cdot \sqrt{\frac{p_o \cdot T}{p \cdot T_o}} = \frac{H_o}{\sqrt{\dfrac{\rho}{\rho_{L,n}}}} \tag{2}$$

2

in denen

$$H_{o,n} = H_o \cdot \frac{p_o \cdot T}{p \cdot T_o} \text{ den Normbrennwert}$$

(früher: oberer Heizwert)

$H_o$     den Betriebsbrennwert

$d_v$     das Dichteverhältnis des Gases, bezogen auf Luft $= 1$

$\rho$     die Gasbetriebsdichte; $\rho = \rho_n \cdot \dfrac{p \cdot T_o}{p_o \cdot T}$

$\rho_{L,n}$     die Normdichte der Luft bezeichnet.

— In analoger Weise sind der untere Norm-Wobbe-Index $W_{u,n}$ und der untere Betriebs-Wobbe-Index $W_u$ definiert, die sich auf den Norm-Heizwert $H_{u,n}$ (früher: unterer Heizwert) bzw. den Betriebsheizwert $H_u$ beziehen. —

Aus den Beziehungen (1) und (2) ist ersichtlich, daß es möglich sein sollte, den neu gefundenen Zusammenhang zwischen Viskosität und Wobbe-Index zur Heizwert- oder Brennwertbestimmung zu benutzen, wenn die Dichte des Gases bekannt ist bzw. gemessen wird.

Aus diesen Erkenntnissen wurde unter Einbeziehung an sich bekannter Methoden zur Bestimmung der Viskosität sowie der Dichte von Gasen eine Lehre zum technischen Handeln entwickelt, die den Gegenstand dieser Erfindung bildet und die in den Ansprüchen 1 und 2 (Verfahren) sowie in den Ansprüchen 10 – 13 und 15 – 17 (Vorrichtungen) angegeben ist. Weiterbildungen sind in Unteransprüchen gekennzeichnet.

Die Erfindung basiert auf der allgemeinen Beziehung

$$f(H) = f(n, \varrho) \tag{3}$$

aus der die empirisch gefundene allgemeine Formel

$$f(H) = a + b \cdot n^c \cdot \varrho^d \tag{4}$$

als erste Näherung entwickelt wurde, die sich auf bestimmte Betriebsbedingungen (p, T = Konstant) bezieht.

Die in diesen Formeln verwendeten Buchstaben haben folgende Bedeutungen:

$f(H)$     Funktion der Reaktionswärme des Gases, z. B. $H_{o,n}$ $H_u$, $W_{o,n}$, $W_u$ in MJ/m³

a, b, c, d Konstanten, die je nach Bedeutung von $f(H)$ — Brenn- oder Heizwert, oberer oder unterer Wobbe-Index — unterschiedliche Zahlenwerte haben.

n     dynamische Gasviskosität in Ns/m²

$\varrho$     Betriebs-Gasdichte in kg/m³

Die Gültigkeit und der Streubereich der Formel (4) wurde für zahlreiche Fälle mit unterschiedlichen Bedeutungen von $f(H)$ z. B. für $H_{o,n}$, $H_u$, $W_{o,n}$, $W_u$ verifiziert. Dies wird später anhand von Beispielen näher erläutert.

Formel (4) zeigt, daß die Werte von $f(H)$ insbesondere durch die variablen Größen n und $\varrho$ charakterisiert sind. Die Bestimmung dieser Größen kann grundsätzlich mit beliebigen Methoden erfolgen. — Die zur Zeit bekannten Methoden erfordern zum Teil einen erheblichen Aufwand. —

Hier sollen erfindungsgemäß nur Bestimmungsmethoden betrachtet werden, denen die im folgenden aufgeführten physikalischen Gesetzmäßigkeiten bzw. Beziehungen zugrunde liegen.

Für den Druckabfall $\Delta$ p zwischen Eintritts- und Austrittsseite eines Rohres, das von einem Medium durchströmt wird, gilt bei laminarer Strömung (Re ≤ 2300) nach dem Hagen-Poiseulle'schen Gesetz folgende Beziehung

$$\Delta p_l = \frac{32 \cdot l \cdot u_l \cdot n}{d_h^2} \tag{5}$$

deren Symbole folgende Bedeutung haben:

Re     Reynoldszahl

$\Delta$ $p_l$     Druckdifferenz bei laminarer Strömung in N/m²

$u_l$     mittlere Gasgeschwindigkeit in m/s

l     Rohrlänge in m

3

$d_h$     hydraulischer Rohrdurchmesser in m

n     dynamische Gasviskosität in $Ns/m^2$

Bei konstanten geometrischen Bedingungen ist $u_l = \frac{V_l}{F}$, wobei $V_l$ den Volumenstrom und F den Querschnitt des Rohres bezeichnen.

Daraus ergibt sich mit Gleichung (5):

$$n \sim \frac{\Delta p_l}{V_l} \tag{6}$$

Für die Geschwindigkeit $u_t$ des mit turbulenter Strömung durch eine Düse fließenden Gases folgt aus dem Energieerhaltungssatz

$$u_t = k \cdot g \cdot e \cdot \sqrt{\frac{2 \Delta p_t}{\rho}} \tag{7}$$

In dieser Formel bedeuten die bisher nicht verwendeten Symbole:

k     Düsenkonstante

g     Durchflußzahl

e     Expansionszahl

$\Delta p_t$     Druckdifferenz bei turbulenter Strömung in $N/m^2$.

Aus Formel (7) ergibt sich für konstante geometrische Verhältnisse die Beziehung

$$\rho \sim \frac{\Delta p_t}{V_t^2} \tag{8}$$

Die (meßbaren) Druckdifferenzen $\Delta p_l$ und $\Delta p_t$ sind demgemäß ein Maß für die Gasviskosität n bzw. die Gasdichte $\rho$. Die vorstehend genannten Beziehungen bzw. physikalischen Gesetze werden bei der Verifizierung der vorliegenden Erfindung unter Kombination der Beziehungen (4), (6) und (8) benutzt.

$$f(H) = a + b \cdot \left(\frac{\Delta p_l}{V_l}\right)^c \cdot \left(\frac{\Delta p_t}{V_t^2}\right)^d \tag{9}$$

Dabei bestimmen physikalische Eigenschaften in den Anordnungen zum Teil die Konstanten a, b, c, d der Formel (9). Das Ausgangssignal dieser Anordnungen approximiert also mehr oder weniger genau die Formel (4) und liefert so ein Maß für f (H).

Zur Durchführung des erfindungsgemäßen Verfahrens muß in jedem Fall die dynamische Gasviskosität bestimmt werden, die ein Maß für den Norm-Wobbe-Index des Gases ist. Diese Messung erfolgt in an sich bekannter Weise mit Hilfe eines Strömungswiderstandes, der eine laminare Gasströmung bewirkt und der im folgenden als Laminarwiderstand bezeichnet wird.

Nach Beziehung (6) gibt es dazu drei Möglichkeiten:

1. Der Druckabfall über dem Laminarwiderstand wird konstant gehalten, und die Durchflußmenge wird gemessen.
2. Der Druckabfall über dem Laminarwiderstand wird gemessen, und die Durchflußmenge wird konstant gehalten.
3. Der Druckabfall über dem Laminarwiderstand und die Durchflußmenge werden gleichzeitig gemessen.

Wenn der Heiz- oder Brennwert ermittelt werden soll, ist es außerdem erforderlich, die Dichte des Gases zu ermitteln. Diese Messung erfolgt in an sich bekannter Weise mit Hilfe eines Strömungswiderstandes, der eine turbulente Gasströmung bewirkt und der im folgenden als Turbulenzwiderstand bezeichnet wird.

Nach Beziehung (8) gibt es dafür grundsätzlich die gleichen drei Möglichkeiten, die oben für den Laminarwiderstand angegeben sind.

Durch Kombination von Laminar- und Turbulenzwiderständen, deren Druckabfälle und Volumenströme gemessen und/oder geregelt werden, können je nach Anwendungszweck die Ausgangsgrößen der Meßanordnungen direkt oder nach Umrechnung als Steuer- und/oder Anzeigesignale verwendet werden.

Im folgende    rden zu einigen beispielsweise ausgewählten Meßanordnungen die zugehörenden physikalischen Zusammenhänge ohne Berücksichtigung der Einflüsse von Temperatur und Gesamtdruck dargestellt. Hierdurch wird eine unnötige Komplizierung der Formeln, durch die die Übersichtlichkeit leiden würde, vermieden. Die Möglichkeiten zur Kompensation von Temperatur und Absolutdruckeinfluß auf die Ergebnisse werden im Anschluß an die Beschreibung der Vorrichtungen dargestellt.

Die benötigten Stoffwerte der Einzelgaskomponenten (für $\varrho_n$, n, $H_{o,n}$, $H_{u,n}$, $W_{o,n}$, $W_{u,n}$) und einiger Gasgemische sind maßgeblichen Normen und Handbüchern entnommen /1 bis 4/. Für die Berechnung der Stoffwerte weiterer Gemische sind die in /4/ wiedergegebenen Formeln zugrunde gelegt. In Tabelle 1 sind beispielhaft für 4 Gasqualitäten der Erdgasfamilie nach /5/ Zusammensetzungen und Stoffwerte angegeben. Im folgenden dient Erdgas H (siehe Tabelle 1) als Bezugsgas (Index b), das heißt, das Verhalten der durch eine Anordnung strömenden Gase wird im Vergleich zum Verhalten von Erdgas H betrachtet. Damit entfallen sonstige dimensionsbehaftete Konstanten.

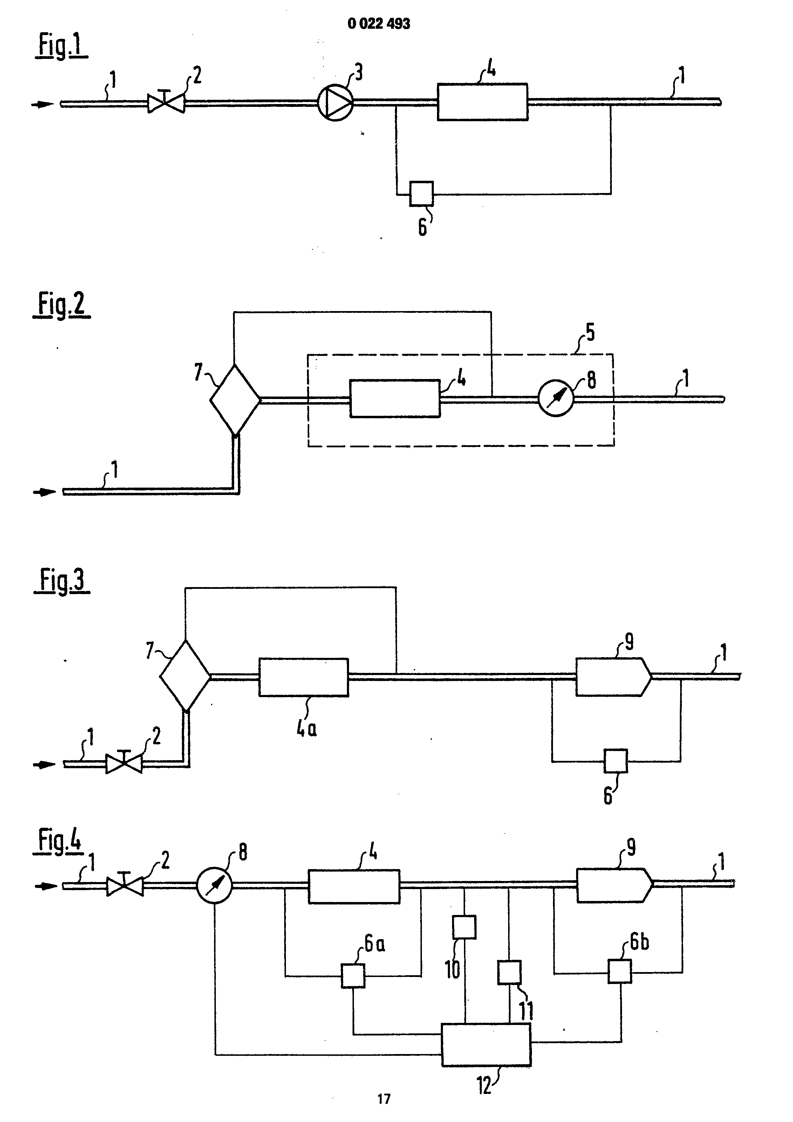

Zur Verdeutlichung des erfindungsgemäßen Verfahrens werden nachstehend unter Bezugnahme auf die Fig. 1 bis 7 verschiedene beispielsweise Vorrichtungen und deren Wirkungsweisen näher beschrieben. In den Figuren sind die einzelnen Vorrichtungsteile schematisch dargestellt. Gleiche Vorrichtungsteile sind in allen Figuren mit übereinstimmenden Ziffern bezeichnet worden.

Wenn nur der Wobbe-Index eines Gases gemessen werden soll, genügt es, eine aus Volumenstrom-Meß- oder -Dosiereinrichtung und Laminarwiderstand bestehende Meßeinrichtung zu benutzen und entweder den Volumenstrom oder den Differenzdruck in bekannter Weise konstant zu halten, während die andere Größe gemessen wird. Eine solche Meßanordnung ist beispielsweise schematisch in Fig. 1 dargestellt.

Das Gas strömt in Pfeilrichtung durch die Gasleitung 1, die von einer nicht dargestellten Hauptgasleitung abzweigt, und in der ein Druckminderer 2 vor der aus Volumenstrom-Dosiereinrichtung 3, Laminarwiderstand 4 und Differenzdruckmeßeinrichtung 6 bestehenden Meßanordnung angebracht ist. Die Volumenstrom-Dosiereinrichtung 3 sorgt für einen konstanten Volumenstrom des Gases. Der gemessene Differenzdruck, der bei konstanter Temperatur oder mit geeigneter Temperaturkompensation ein Maß für den Norm-Wobbe-Index des momentan fließenden Gases ist, kann in bekannter Weise direkt beziehungsweise nach pneumatischer Verstärkung oder nach Umwandlung in elektrische Impulse auf Anzeige und/oder Steuergeräte übertragen werden. Das Gas kann nach Durchströmen der Meßanordnung wieder der Hauptgasleitung oder einer Gasverbrauchseinrichtung zugeführt werden. Die zuletzt genannte Methode empfiehlt sich beispielsweise bei industriellen Gasverbrauchseinrichtungen, bei denen die so zugeführten Mengen im Vergleich zum Bedarf zu vernachlässigen sind.

Das Signal der Differenzdruck-Meßeinrichtung 6 ist linear abhängig vom Norm-Wobbe-Index. Seine Größe verringert sich bei steigendem Norm-Wobbe-Index.

Soll das Signal der Meßeinrichtung den Betriebs-Wobbe-Index wiedergeben, so ist eine Temperatur- und Absolutdruck-(Barometerstand-) Kompensation erforderlich.

Möglichkeiten hierzu werden im Anschluß an die Beschreibung der Vorrichtungen aufgezeigt.

Für die Meßanordnung gemäß F i g. 1 gilt:

$$\frac{\varDelta p_l}{\varDelta p_{l,b}} = \frac{n}{n_b} \text{ bzw.} \tag{10}$$

$$n = \frac{n_b}{\varDelta p_{l,b}} \cdot \varDelta p_l \tag{11}$$

Da $n_b$ und $\varDelta p_{l,b}$ bekannt und konstant sind, ist das Ausgangssignal $\varDelta p_e$ direkt ein Maß für die Zähigkeit n des unbekannten Gases, die ihrerseits aus den eingangs genannten Gründen ein hinreichend genaues Maß für Wobbe-Indizes ist. Der Einfluß der Gasdichte wird hier nicht berücksichtigt, das heißt die Konstante d nach Formel (4) ist gleich Null. Anordnungen, mit denen der Brenn- oder Heizwert bestimmt werden soll, müssen zusätzlich mindestens einen Turbulenzwiderstand enthalten, damit die Dichte des Gases in das Ausgangssignal eingehen kann, und der Ausdruck $\varrho^d$ in Formel (4) bzw. (12) einen von 1 verschiedenen Wert annimmt.

Durch Einsetzen von Formel (11) in Formel (4) ergibt sich demnach für den Zusammenhang von $\varDelta p_e$ und $W_{u,n}$

$$W_{u,n} = a + b \cdot \left(\frac{n_b}{\varDelta p_{l,b}} \cdot \varDelta p_l\right)^c \cdot 1 \text{ bzw.} \tag{12}$$

$$W_{u,n} = a + b \cdot \left(\frac{n_b}{\varDelta p_{l,b}}\right)^c \cdot \varDelta p_l^c \tag{13}$$

Die Konstanten a, b und c gemäß Formel (13) haben z. B. für die Anordnung gemäß F i g. 1 zur Bestimmung von $W_{u,n}$ in $MJ/m^3$ die Werte:

0 022 493

a = 199,1
b = −74 211
c = 0,542

Eine andere Meßeinrichtung, die ebenfalls ein Signal für den Norm-Wobbe-Index liefert, ist in Fig. 2 dargestellt. Hier wird das Gas durch eine analoge Gasleitung 1 in die Meßanordnung geleitet, die aus einem Laminarwiderstand 4, dessen Druckabfall mit Hilfe des Differenzdruckreglers 7 konstant gehalten wird, und einer Volumenstrom-Meßeinrichtung 8 besteht. Die Temperaturen des Laminarwiderstandes 4 und der Volumenstrom-Meßeinrichtung 8 werden gemeinsam durch Thermostat 5 konstant gehalten. Statt dessen ist es möglich, die notwendige Kompensation des Temperatureinflusses auf das System allein durch Thermostatisierung eines Teiles des Laminarwiderstandes (4) zu erreichen. —

Bei dieser Anordnung ist die Anzeige beziehungsweise das von der Volumenstrom-Meßeinrichtung 8 abgegebene Signal ein Maß für den Norm-Wobbe-Index, wobei sich hier bei steigendem Norm-Wobbe-Index die strömende Gasmenge vergrößert, das heißt das Signal ändert sich in gleichem Sinne bzw. mit gleichem Vorzeichen wie der Norm-Wobbe-Index.

Diese Anordnung kann ohne Änderung ihrer Wirkungsweise so modifiziert werden, daß die Volumenstrom-Meßeinrichtung 8 in Strömungsrichtung vor dem Laminarwiderstand 4, dessen Differenzdruck konstant gehalten wird, angeordnet ist.

Für eine Wiedergabe des Betriebs-Wobbe-Indexes benötigt man wie zu Fig. 1 angegeben, eine Temperatur- und Absolutdruck-Kompensation.

Für die Meßanordnung gemäß Fig. 2 gilt:

$$\frac{V_l}{V_{l,b}} = \frac{n_b}{n} \text{ bzw.} \tag{14}$$

$$n = n_b \cdot V_{l,b} \cdot \frac{1}{V_l} \tag{15}$$

Analog zur Formel (13) ergibt sich durch Einsetzen von Formel (15) in Formel (4) für den Zusammenhang von $V_l$ und $W_{o,n}$

$$W_{o,n} = a + b \cdot \left(n_b \cdot V_{l,b}\right)^c \cdot \left(\frac{1}{V_l}\right)^c \tag{16}$$

Die Konstanten a, b und c gemäß Formel 16 haben zum Beispiel für die Anordnung gemäß Fig. 2 zur Bestimmung von $W_{o,n}$ in MJ/m$^3$ die Werte:

a = 113,8
b = −7,216 · 10$^8$
c = 1,425

Mit der in Fig. 3 dargestellten Vorrichtung wird der Betriebsheizwert oder der Betriebsbrennwert eines Gases direkt gemessen, da bei dieser Anordnung außer dem Laminarwiderstand 4 ein Turbulenzwiderstand 9 im Gasstrom angeordnet ist. Bei dieser Vorrichtung wird der Druckabfall über dem Laminarwiderstand 4 mit Hilfe des Differenzdruckreglers 7 konstant gehalten, während der Druckabfall über Turbulenzwiderstand 9 gemessen wird. Eine geeignete Temperaturkompensation bewirkt, daß der Temperaturgang des bei 6 gemessenen Differenzdruckes gleich dem des Betriebsheizwertes ist. Wird ein Signal für den Norm-Heizwert oder Brennwert gewünscht, so ist eine entsprechende Temperatur- und Absolutdruck-Kompensation erforderlich.

Die in Fig. 3 dargestellte Meßanordnung eignet sich wegen der einfachen Ausführungsform unter anderem besonders gut für die Umwandlung der Volumenmessung einer Gasuhr in eine Energiemengenmessung und wegen der exakten Wiedergabe der Abhängigkeit des Betriebsheizwertes von der Gasdichte für direkte Messungen unter erhöhtem Druck.

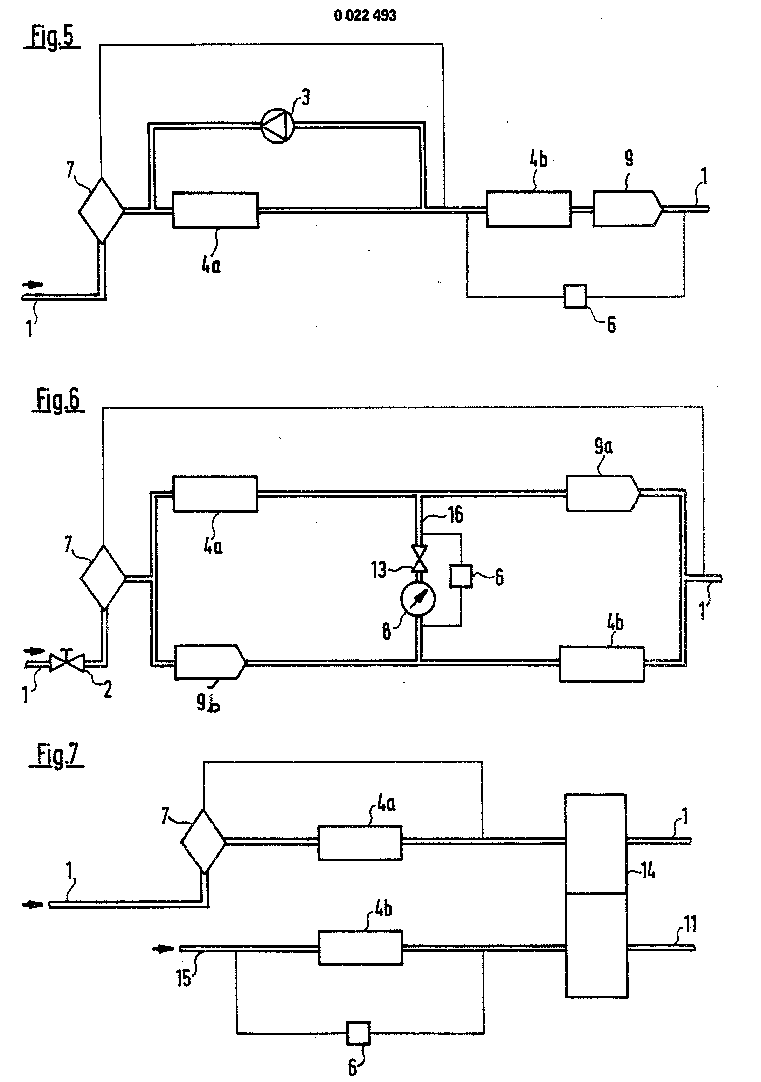

Fig. 4 zeigt eine Meßanordnung, bei der in der Gasleitung 1 hinter einem Druckminderer 2 eine Volumenstrom-Meßeinrichtung 8, beispielsweise eine Gasuhr, ein Laminarwiderstand 4 und ein Turbulenzwiderstand 9 sowie eine Temperatur-Meßeinrichtung 10 und Absolutdruck-Meßeinrichtung 11 angeordnet sind. Über beiden Strömungswiderständen wird der Druckabfall mit entsprechenden Meßeinrichtungen 6a, 6b gemessen. Die an den Instrumenten 8, 6a, 6b, 10 und 11 gemessenen Werte werden in bekannter Weise, zum Beispiel nach Umwandlung in elektrische Impulse, zu einem Kleincomputer 12 übertragen, der die Meßwerte auf Anzeige bzw. Aufzeichnungseinrichtungen überträgt und/oder Impulse auf Regel- beziehungsweise Steuerorgane in der Hauptgasleitung überträgt.

Durch Verwendung des Kleincomputers 12 können bei dieser Anordnung nicht nur die unmittelbar

6

gemessenen Werte (Gasvolumenstrom, Differenzdrücke, Temperatur- und Absolutdruck des Gases) sondern auch abgeleitete Werte angezeigt beziehungsweise auf schreibende Instrumente übertragen oder als Regelimpulse abgegeben werden, zum Beispiel Wobbe-Indizes, Heizwert, Brennwert sowie Gasdichte und Viskosität, und zwar sowohl für Betriebs- wie für Normbedingungen.

Diese Art der Meßanordnung eignet sich insbesondere, wenn eine Mehrzahl von Gasverbrauchseinrichtungen, die individuell gesteuert werden müssen, der Meßanordnung nachgeschaltet sind. Die Anordnung eignet sich gleichfalls zum Einbau in Meßstationen von Hochdruck-Gasfernleitungen, in denen der (Wärme)-Mengenfluß fortlaufend registriert wird.

Die in Fig. 5 dargestellte Meßanordnung unterscheidet sich von der in Fig. 3 beschriebenen dadurch, daß im Gasweg ein zweiter Laminarwiderstand 4b angeordnet ist und daß mit Hilfe der Volumenstrom-Dosiereinrichtung 3 eine bestimmte Gasmenge hinter dem Laminarwiderstand 4a aus der Gasleitung abgezweigt wird, die dieser vor dem Laminarwiderstand 4a wieder zugeführt wird, das heißt eine bestimmte Gasmenge wird auf diese Weise im Kreislauf geführt. Dem Laminarwiderstand 4a sind der Laminarwiderstand 4b und ein Turbulenzwiderstand 9 im Gasweg nachgeschaltet. Der Druckabfall über die beiden zuletzt genannten Strömungswiderstände wird gemessen. Je nach Abstimmung der Widerstände zueinander ist bei konstanter Temperatur und konstantem Barometerstand oder mit geeigneter Temperatur- und Absolutendruckkompensation der an der Differenzdruck-Meßeinrichtung 6 gemessene Druckabfall proportional dem Norm-Wobbe-Index (bei überwiegendem Einfluß des Laminarwiderstandes 4b) oder proportional dem Betriebsbrenn- oder Heizwert (bei überwiegendem Einfluß des Turbulenzwiderstandes 9).

Signale für Betriebs-Wobbe-Indizes oder Normbrenn- oder -heizwerte setzen andere geeignete Kompensationen der Einflüsse von Absolutdruck und Temperatur voraus.

Bei der in Fig. 6 dargestellten Meßanordnung verzweigt sich die Gasleitung 1 in zwei parallele Leitungsabschnitte, in denen je ein Laminarwiderstand 4 und ein Turbulenzwiderstand 9 angeordnet sind, wobei sich als erster Widerstand im einen Fall ein Laminar- und im anderen Fall ein Turbulenzwiderstand in dem Leitungsabschnitt befindet. Die beiden Leitungsabschnitte vereinigen sich hinter den Meßgeräten wieder zu einer gemeinsamen Leitung und der Differenzdruckregler 7 hält den Druckabfall über dem Gesamtbereich aller Strömungswiderstände konstant, wie aus der Zeichnung ersichtlich ist. Die Messung eines Druckabfalles oder eines Gasvolumenstromes erfolgt bei dieser Meßanordnung mit Hilfe entsprechender Einrichtungen 6 und 8, die in einer Brückenleitung 16 zwischen den beiden parallelen Leitungsabschnitten — zwischen den Strömungswiderständen — angeordnet sind und die mit Hilfe des Absperrhahnes 13 wechselweise beaufschlagt werden können.

Der Vorteil dieser Anordnung besteht darin, daß die Anzeige so einstellbar ist, daß bei einer vorbestimmten Gasqualität ein Signal »Null« angezeigt wird. Bei Änderung der Gasqualität ist aus dem Signal sofort ersichtlich, ob eine positive oder negative Änderung eingetreten ist. Außerdem sind die von der Null-Stellung abweichenden Signale zu Beginn der Abweichung je nach Meßgerät relativ groß, da die Kurve der Abweichungen insbesondere bei einfachen Meßgeräten nach zunächst starkem Anstieg asymptotisch verläuft.

Mit Hilfe der Meßanordnung, die in Fig. 7 dargestellt ist, kann ein gemessener Brenngas-Volumenstrom auf den Volumenstrom eines anderen Mediums, vorzugsweise Luft, auf folgende Weise übertragen werden: Das Brenngas strömt durch Gasleitung 1, in der sich ein Laminarwiderstand 4a befindet, dessen Druckabfall mit Hilfe des Differenzdruckreglers 7 konstant gehalten wird, und anschließend durch eine Volumenstrom-Übertragungseinrichtung 14, welche durch Leitung 15 eine der Brenngasmenge entsprechende Luftmenge ansaugt.

In Leitung 15 ist vor der Volumenstrom-Übertragungseinrichtung 14 ein Laminarwiderstand 4b angeordnet, dessen Druckabfall mit der Differenzdruck-Meßeinrichtung 6 gemessen wird. Diese Meßanordnung bietet den Vorteil, daß der bei 6 gemessene Druckabfall unabhängig von der Temperatur ist und dementsprechend unmittelbar ein Signal für den Norm-Wobbe-Index darstellt, das heißt eine Temperaturkompensation ist bei dieser Meßanordnung überflüssig.

Die Laminarwiderstände bestehen zweckmäßigerweise aus einem oder mehreren Rohren mit kreis- oder ringförmigem Querschnitt, insbesondere Kapillarrohren, deren Verhältnis von Länge zu hydraulischem Durchmesser mindestens 20 : 1, vorzugsweise 200 bis 2000 : 1 beträgt. Die Rohren sind am Eingang abgerundet und am Ausgang konisch erweitert. Der Winkel zwischen Konus und Rohrachse soll kleiner als 20°, zweckmäßig kleiner als 10° sein. Volumenströme und Querschnitte der Rohre sind so abgestimmt, daß die Reynoldszahl in keinem Fall den Wert 2300 überschreitet. Hierdurch entspricht der zwischen Anfang und Ende der Kapillaren entstehende Differenzdruck weitestgehend einer rein laminaren Strömung.

Die Turbulenzwiderstände bestehen zweckmäßigerweise aus dünnen Lochblenden, deren Verhältnis von Lochdurchmesser zu Länge größer als 2 : 1 ist und vorzugsweise 5 : 1 bis 20 : 1 beträgt und die nahezu frei von Reibungswiderständen sind. Hierdurch ergibt sich vor und hinter der Blende ein Differenzdruck, der praktisch nur durch die Beschleunigung des Gases an der Blende entsteht und demnach auch bei Strömungen, deren Reynoldszahl Re kleiner als 2300 ist, also auf eine laminare Strömung hinweist, noch weitgehend einer rein turbulenten Strömung entspricht.

Die Temperaturkompensation kann zum Beispiel durch Thermostatisierung mindestens eines Teiles des im Strömungsweg ersten Laminarwiderstandes oder durch Veränderung des Differenzdruckes

**0 022 493**

eines Differenzdruckreglers erfolgen.

Wird die Temperaturkompensation durch eine Thermostatisierung eines Teiles des ersten *Laminarwiderstandes vorgenommen, so ist es zweckmäßig, hierfür das Ende des Widerstandes zu benutzen, so daß der übrige Teil des Widerstandes* Umgebungstemperatur behält. Hinter dem Widerstand sorgt ein Wärmetauscher dafür, daß die übrigen Teile der Anordnung ebenfalls Umgebungstemperatur behalten.

Bei Anordnungen mit Differenzdruckregler an einem Strömungswiderstand kann die Temperatur- und Absolutdruckkompensation durch Veränderung der Belastung der Membran des Druckreglers in bekannter Weise erfolgen.

Sofern bei den vorbeschriebenen Anordnungen nur ein Wert zu messen ist, kann dieser in bekannter Weise direkt oder nach Umwandlungen in andere mechanische (z. B. Hub) oder elektrische Signale zum Beispiel zur Konstanthaltung des Energiestromes für eine Gasverbrauchseinrichtung oder zur Umwandlung der Gasmengenanzeige einer Gasuhr in eine Energiestromanzeige benutzt werden. Sind mehrere Werte zu messen, ist die Zwischenschaltung einer Recheneinrichtung erforderlich, die mehrere gemessene Werte zu einem Signal zusammenfassen kann.

Für die Umwandlung der Volumenstromanzeige V einer Gasuhr in einen Energiestrom Q ist die Beziehung

$$Q = H_o = \cdot V \tag{17}$$

maßgebend. Das von einer Anordnung für die Gasbrennwert-Bestimmung vorliegende Signal verstellt an der Gasuhr beispielsweise ein vor der Anzeige eingebautes Getriebe oder wirkt auf den Hub der Bälge in der Gasuhr ein, so daß $H_o \cdot V$ konstant bleibt.

Für die Konstanthaltung der Wärmebelastung Q einer Gasverbrauchseinrichtung gilt bei konstanten geometrischen Bedingungen, die aus den Beziehungen (1), (7) und (17) ableitbare Beziehung (18), wenn die Gasdosierung über Düsen erfolgt,

$$Q = k \cdot F \cdot \sqrt{\Delta p} \cdot W_u \tag{18}$$

Ändert sich der Wobbe-Index $W_u$, so bleibt Q konstant, wenn man durch Zumischung von Inertgasen bzw. Gasen mit höherem Heizwert $W_u$ oder durch Einwirkung auf den Differenzdruck $\Delta p$ an den Gasdüsen $\sqrt{\Delta p} \cdot W_u$ oder durch Einwirkung auf den freien Querschnitt F, der Gasdüse beziehungsweise auf den Querschnitt einer zusätzlichen Gasdüse $F \cdot W_u$ konstant hält.

/1/ DIN 51 850, Entwurf September 1978
/2/ DIN 1871, Februar 1978
/3/ D'Ans Lax, Taschenbuch für Chemiker und Physiker, 1967
/4/ VDI-Wärmeatlas, Auflage 1974
/5/ DVGW-Richtlinie G 260, Januar 1973

8

**0 022 493**

Zusammensetzung und Eigenschaften von Erdgasen in den Grenzen der DVGW-Richtlinie G 260

| | Einheit | $H_{max.}$ | $H$ | $L$ | $L_{min.}$ |
|---|---|---|---|---|---|
| Gasanalyse: | | | | | |
| $CH_4$ | Vol. % | 83,2 | 92,9 | 81,8 | 77,3 |
| $C_2H_6$ | Vol. % | 10,0 | 3,0 | 2,8 | 2,8 |
| $C_2H_4^+$ | Vol. % | — | — | — | — |
| $C_3H_8$ | Vol. % | 4,3 | 1,5 | 0,4 | 0,3 |
| $C_4H_{10}$ | Vol. % | 1,0 | 0,4 | 0,1 | 0,1 |
| $C_5^+$ | Vol. % | 0,2 | 0,2 | 0,1 | 0,1 |
| $H_2$ | Vol. % | — | — | — | — |
| $CO$ | Vol. % | — | — | — | — |
| $CO_2$ | Vol. % | 1,0 | 1,0 | 1,0 | 1,0 |
| $N_2$ | Vol. % | 0,3 | 1,0 | 13,8 | 18,4 |
| $O_2$ | Vol. % | — | — | — | — |
| Brennwert $H_{o,n}$ | MJ/m$^3$ | 46,19 | 41,49 | 35,25 | 33,36 |
| Heizwert $H_{u,n}$ | MJ/m$^3$ | 41,85 | 37,47 | 31,80 | 30,09 |
| Dichteverh. (Luft = 1) $d_o$ | — | 0,678 | 0,609 | 0,643 | 0,661 |
| Viskosität/20°C $n \cdot 10^5$ | Ns/m$^2$ | 10,46 | 10,83 | 11,96 | 12,32 |
| Viskosität/50°C $n \cdot 10^6$ | Ns/m$^2$ | 11,39 | 11,78 | 12,97 | 13,35 |
| ob. Wobbeindex $W_{o,n}$ | MJ/m$^3$ | 56,10 | 53,17 | 43,96 | 41,03 |
| unt. Wobbeindex $W_{u,n}$ | MJ/m$^3$ | 50,83 | 48,01 | 39,66 | 37,01 |
| $W_u/W_{ub}$ | — | 1,059 | 1,000 | 0,826 | 0,771 |
| $H_o/H_{o,b}$ | — | 1,113 | 1,000 | 0,850 | 0,804 |
| ob. Wobbeindix $W_{o,n}$ | kcal/m$^3$ | 13 400 | 12 700 | 10 500 | 9 800 |

**Patentansprüche**

1. Verfahren zur verbrennungslosen Messung und/oder Regelung der Wärmemengenzufuhr zu Gasverbrauchseinrichtungen, insbesondere Erdgasverbrauchseinrichtungen, dadurch gekennzeichnet, daß das Brenngas bzw. ein Teilstrom des Brenngases durch mindestens einen Strömungswiderstand, der eine laminare Strömung und einen Druckabfall bewirkt, geleitet wird und daß der Gasvolumenstrom und/oder der Druckabfall über mindestens einem Strömungswiderstand gemessen wird, wobei die Druckabfälle und/oder Volumenströme, die nicht gemessen werden, konstant gehalten werden, daß der Temperatureinfluß auf das System kompensiert wird, und daß die gemessene(n) Größe(n) gemäß der Beziehung (4)

$$f(H) = a + b \cdot n^c \cdot \varrho^d$$

ein Maß für den Wärmeinhalt des fließenden Gases ist bzw. sind, wobei f (H) ein Brenn- oder Heizwert oder ein Wobbe-Index ist, n die dynamische Gasviskosität, $\varrho$ die Betriebsgasdichte und a, b, c und d Konstanten sind, und daß daraus ein Meß- und/oder Regelsignai abgeleitet wird.

2. Verfahren zur verbrennungslosen Messung und/oder Regelung der Wärmemengenzufuhr zu Gasverbrauchseinrichtungen, insbesondere Erdgasverbrauchseinrichtungen, dadurch gekennzeichnet, daß das Brenngas beziehungsweise ein Teilstrom des Brenngases durch mindestens zwei hintereinander geschaltete und/oder parallel geschaltete Strömungswiderstände geleitet wird, von denen mindestens einer eine laminare und ein anderer eine turbulente Strömung und entsprechende Druckabfälle bewirken, und daß der Druckabfall über mindestens einen Strömungswiderstand konstant gehalten und über dem bzw. den anderen Strömungswiderstand (-widerständen) gemessen wird oder daß der Druckabfall über allen gemeinsam konstant gehalten und über mindestens einem Strömungswiderstand gemessen wird, daß der Temperatureinfluß auf das System kompensiert wird, und daß die gemessene(n) Größe(n) gemäß der Beziehung (4)

$$f(H) = a + b \cdot n^c \cdot \varrho^d$$

ein Maß für den Wärmeinhalt des fließenden Gases ist beziehungsweise sind, wobei f (H) ein Brenn- oder Heizwert oder ein Wobbe-Index ist, n die dynamische Gasviskosität, $\varrho$ die Betriebsgasdichte und a, b, c und d Konstanten sind, und daß daraus ein Meß- und/oder Regelsignal abgeleitet wird.

3. Verfahren nach Anspruch 1, dadurch gekennzeichnet, daß zur Kompensation des Temperatureinflusses der gemessene Gasvolumenstrom oder der von einem Strömungswiderstand erzeugte Druckabfall dem Betrage nach auf eine analoge Meßeinrichtung übertragen wird, die von einem anderen Gas, vorzugsweise Luft, durchströmt wird.

4. Verfahren nach Anspruch 2, dadurch gekennzeichnet, daß das Gas außer durch laminare und turbulente Strömungswiderstände auch durch eine Volumenstrom-Meß- oder -Dosiereinrichtung geleitet wird, mit welcher ein Volumenstrom des Gases gemessen oder konstant gehalten wird.

5. Verfahren nach Anspruch 2 oder 4, dadurch gekennzeichnet, daß das Gas bzw. ein Teilstrom des Gases durch zwei parallel angeordnete Gruppen von je zwei hintereinander geschalteten Strömungswiderständen geleitet wird, von denen jeweils einer eine laminare und der zweite eine turbulente Gasströmung bewirkt, und daß bei der einen Gruppe die laminare Strömung im ersten und bei der anderen Gruppe die laminare Strömung im letzten Strömungswiderstand bewirkt wird, daß der Druckabfall über den beiden Gruppen gemeinsam konstant gehalten wird, und daß der Druckabfall oder der Volumenstrom des Gases in einer zwischen den beiden Gruppen zwischen den laminaren und turbulenten Strömungswiderständen angeordneten Brücke gemessen wird.

6. Verfahren nach einem der Ansprüche 2, 4 oder 5, dadurch gekennzeichnet, daß die gemessenen Druckdifferenzen und Volumenströme sowie ggf. Temperatur- und Absolutdruck-Meßwerte — um deren Einflüsse auf das System zu kompensieren — zu einem Kleincomputer übertragen werden, der diese zu einem Regel- oder Steuerimpuls umwandelt und/oder errechnete Werte auf Anzeige und/oder Registriergeräte überträgt.

7. Verfahren nach einem der Ansprüche 1 bis 5, dadurch gekennzeichnet, daß außer dem Temperatureinfluß auch der Absolutdruckeinfluß auf das System kompensiert wird.

8. Verfahren nach einem der Ansprüche 1 bis 5, dadurch gekennzeichnet, daß zur Kompensation des Temperatureinflusses der im Strömungsweg erste Laminarwiderstand mindestens teilweise thermostatisiert wird.

9. Verfahren nach Anpruch 7, dadurch gekennzeichnet, daß zur Kompensation des Temperatur- und/oder Absolutdruckeinflusses die Membran des Differenzdruckreglers beeinflußt wird.

10. Anordnung zur Durchführung des Verfahrens nach Anspruch 1, unter Verwendung einer zur Gasverbrauchseinrichtung führenden Gasleitung, dadurch gekennzeichnet, daß in der Gasleitung (1) eine Volumenstrom-Dosiereinrichtung (3) und mindestens ein Laminarwiderstand (4) angeordnet sind, daß der Laminarwiderstand (4) mit einer Differenzdruck-Meßeinrichtung (6) verbunden ist, und daß die Anordnung mit einer Einrichtung zur Kompensation des Temperatureinflusses auf das System versehen ist.

11. Anordnung zur Durchführung des Verfahrens nach Anspruch 1, unter Verwendung einer zur Gasverbrauchseinrichtung führenden Gasleitung dadurch gekennzeichnet, daß in der Gasleitung (1) eine Volumenstrom-Meßeinrichtung (8) und mindestens ein Laminarwiderstand (4) sowie mindestens ein Differenzdruckregler (7) zur Konstanthaltung des Druckabfalles am Laminarwiderstand (4) angeordnet sind und daß die Anordnung mit einer Einrichtung zur Kompensation des Temperatureinflusses versehen ist.

12. Anordnung zur Durchführung des Verfahrens nach Anspruch 2, unter Verwendung einer zur Gasverbrauchseinrichtung führenden Gasleitung, dadurch gekennzeichnet, daß in der Gasleitung (1) mindestens je ˙. Laminarwiderstand (4) und ein Turbulenzwiderstand (9) angeordnet sind, und daß mindestens einer dieser Strömungswiderstände mit einer Differenzdruck-Meßeinrichtung (6) verbunden ist, daß die anderen Strömungswiderstände mit einem Differenzdruckregler (7) verbunden sind und daß die Anordnung mit einer Einrichtung zur Kompensation des Temperatureinflusses versehen ist.

13. Anordnung zur Durchführung des Verfahrens nach Anspruch 2, unter Verwendung einer zur Gasverbrauchseinrichtung führenden Gasleitung, dadurch gekennzeichnet, daß in der Gasleitung (1) mindestens je ein Laminarwiderstand (4) und ein Turbulenzwiderstand (9) angeordnet sind, und daß alle Strömungswiderstände gemeinsam mit einem Differenzdruckregler (7) verbunden sind, daß mindestens ein Strömungswiderstand mit einer Differenzdruck-Meßeinrichtung (6) verbunden ist und daß die Anordnung mit einer Einrichtung zur Kompensation des Temperatureinflusses versehen ist.

14. Anordnung nach Anspruch 12 oder 13, dadurch gekennzeichnet, daß zusätzlich in der Brenngasleitung (1) eine Volumenstrom-Meß- oder -Dosiereinrichtung (8 oder 3) angeordnet ist.

15. Anordnung zur Durchführung des Verfahrens nach Anspruch 3, unter Verwendung einer zur Gasverbrauchseinrichtung führenden Gasleitung, dadurch gekennzeichnet, daß in der Gasleitung (1) ein Laminarwiderstand (4a), der mit einem Differenzdruckregler (7) verbunden ist sowie eine Volumenstrom-Übertragungseinrichtung (14) angeordnet sind, und daß die Volumenstrom-Übertragungseinrichtung (14) außerdem mit einer Bezugsgasleitung (15) verbunden ist, in der sich ein Laminarwiderstand (4b), der mit einer Differenzdruck-Meßeinrichtung (6) verbunden ist, befindet.

16. Anordnung zur Durchführung des Verfahrens nach Anspruch 4, unter Verwendung einer zur Gasverbrauchseinrichtung führenden Gasleitung, dadurch gekennzeichnet, daß in der Gasleitung (1) mindestens zwei Laminarwiderstände (4a, 4b) sowie ein Turbulenzwiderstand (9) hintereinander angeordnet sind, und daß eine Volumenstrom-Dosiereinrichtung (3) mit mindestens einem Laminarwiderstand (4a) und einem Differenzdruckregler (7) verbunden ist, und daß ein weiterer Laminarwiderstand (4b) und der Turbulenzwiderstand (9) gemeinsam mit einer Differenzdruck-Meßeinrichtung (6) verbunden sind und daß die Anordnung mit einer Einrichtung zur Kompensation des Temperatureinflusses versehen ist.

17. Anordnung zur Durchführung des Verfahrens nach Anspruch 5, unter Verwendung einer zur Gasverbrauchseinrichtung führenden Gasleitung, dadurch gekennzeichnet, daß in der Gasleitung (1) ein Druckminderer (2) angeordnet ist, und daß die Brenngasleitung (1) danach in Form von zwei parallelen Teilstromleitungen ausgebildet ist, daß in jeder der Teilstromleitungen hintereinander mindestens ein Laminarwiderstand (4a, 4b) und ein Turbulenzwiderstand (9a, 9b) angeordnet sind, und daß in der einen Teilstromleitung der Laminarwiderstand (beziehungsweise die Laminarwiderstände) vor und in der anderen Teilstromleitung der Laminarwiderstand (beziehungsweise die Laminarwiderstände) hinter dem Turbulenzwiderstand angeordnet ist (sind), und daß ein Differenzdruckregler (7) vor und hinter der Aufteilung in die Teilstromleitungen mit der Brenngasleitung (1) verbunden ist, daß die beiden Teilstromleitungen zwischen den Laminar- und Turbulenzwiderständen durch eine Brückenleitung (16) miteinander verbunden sind, in der eine Differenzdruck-Meßeinrichtung (6) und/oder eine Volumenstrom-Meßeinrichtung (8) angeordnet sind, und daß die Anordnung mit einer Einrichtung zur Kompensation des Temperatureinflusses versehen ist.

18. Anordnung nach einem der Ansprüche 12 bis 17, dadurch gekennzeichnet, daß die Differenzdruck-Meßeinrichtungen (6) und die Volumenstrom-Meßeinrichtung (8) mit einem Kleincomputer (12) verbunden sind.

19. Strömungswiderstand nach einem der Ansprüche 10 bis 18, dadurch gekennzeichnet, daß der Laminarwiderstand (4) als Rohr mit kreis- oder ringförmigem Querschnitt oder als entsprechendes Rohrbündel ausgebildet ist, dessen Verhältnis von Länge zu hydraulischem Durchmesser mindestens 20 : 1, vorzugsweise 200 bis 2000 : 1 beträgt, und daß jedes Einzelrohr am Eingang abgerundet und am Ende konisch erweitert ist, wobei die konische Erweiterung einen Winkel zur Rohrachse von kleiner als 20° vorzugsweise kleiner als 10° aufweist.

20. Strömungswiderstand nach einem der Ansprüche 12 bis 19, dadurch gekennzeichnet, daß der Turbulenzwiderstand (9) als Lochblende ausgebildet ist, deren Verhältnis von Lochdurchmesser zu Länge größer als 2 : 1 ist und vorzugsweise 5 : 1 bis 20 : 1 beträgt.

21. Vorrichtung nach einem der Ansprüche 10 bis 20, dadurch gekennzeichnet, daß der im Strömungsweg erste Laminarwiderstand (4) mindestens teilweise von einem Thermostaten (5) umgeben ist.

22. Vorrichtung nach einem der Ansprüche 10 bis 21 dadurch gekennzeichnet, daß die Membran eines Differenzdruckreglers (7) in bekannter Weise mit einer temperatur- und/oder absolutdruckabhängigen Kraft beaufschlagt wird.

## Claims

1. A method of measuring and/or controlling without combustion the heat supply to gas consuming devices, in particular natural gas consuming devices, characterised in that the combustion gas for a branch current of the combustion gas is passed through at least one flow resistance which produces a laminar flow and a pressure drop and that the gas flow rate and/or the pressure drop is measured across at least one flow resistance, the pressure drops and/or flow rates which are not measured being maintained constant, that the effect of temperature on the system is compensated for and that the measured value(s) is/are a measure of the heat content of the flowing gas in accordance with the relationship (4)

$$f(H) = a + b \cdot n^c \cdot \varrho^d$$

whereby f (H) is a calorific or heating value or a Wobbe index, n the dynamic gas viscosity, $\varrho$ the operational gas density and a, b, c and d are constants and that a measuring and/or control signal is derived therefrom.

2. Method of measuring and/or controlling without combustion the heat supply to gas consuming devices, in particular natural gas consuming devices, characterised in that the combustion gas or a branch current of the combustion gas is passed through at least two parallel and/or series connected flow resistances of which at least one produces a laminar and the other a turbulent flow and corresponding pressure drops, and that the pressure drop across at least one flow resistance is held constant and is measured across the other flow resistance (resistances) or that the pressure drop is maintained constant over all of them together and is measured across at least one flow resistance, that the temperature effect on the system is compensated for, and that the measured value(s) is/are a measure of the heat content of the flowing gas in accordance with the relationship (4)

$$f(H) = a + b \cdot n \cdot \varrho^d$$

where f (H) is a calorific or heating value or a Wobbe index, n the dynamic gas viscosity, $\varrho$ the operational gas density and a, b, c and d are constants and that a measuring and/or control signal is derived therefrom.

3. A method as claimed in Claim 1, characterised in that to compensate for the temperature effect the measured gas flow rate or the amount of the pressure drop produced across a flow resistance is transmitted to an analogous measuring device through which another gas, preferably air, flows.

4. A method as claimed in Claim 2 characterised in that in addition to laminar and turbulent flow resistances the gas is also passed through a flow rate measuring or metering device with which a flow rate of the gas is measured or maintained constant.

5. A method as claimed in Claim 2 or 4, characterised in that the gas or a branch current of the gas is passed through two parallel arranged groups of two series connected flow resistances of which one produces a laminar and the other a turbulent gas flow, and that in the one group the laminar flow is produced in the first flow resistance and the other group the laminar flow is produced in the last flow resistance, that the pressure drop across the two groups is maintained constant together, and that the pressure drop or the flow rate of the gas is measured in a bridge arranged between the two groups between the laminar and turbulent flow resistances.

6. A method as claimed in one of Claims 2, 4 or 5, characterised in that the measured pressure differences and flow rates and also optionally temperature and absolute pressure measured values — in order to compensate for their effects on the system — are transmitted to a small computer which converts them into a control or command impulse and/or transmits calculated values to indicating and/or registering devices.

7. A method as claimed in one of Claims 1 to 5, characterised in that in addition to the temperature effect the absolute pressure effect on the system is also compensated for.

8. A method as claimed in one of Claims 1 to 5, characterised in that to compensate for the temperature effect the first laminar resistance in the flow path is at least partly thermostatically temperature controlled.

9. A method as claimed in Claim 7, characterised in that to compensate for the effect of temperature and/or absolute pressure the membrane of the differential pressure controller is controlled.

10. Apparatus for carrying out the method as claimed in Claim 1 using a gas conduit leading to the gas consuming device, characterised in that a flow rate metering device (3) and at least one laminar resistance (4) are arranged in the gas conduit (1), that the laminar resistance (4) is connected to a differential pressure measuring device (6), and that the apparatus is provided with a device for compensating for the effect of temperature on the system.

11. Apparatus for carrying out the method as claimed in Claim 1 using a gas conduit leading to the gas consuming device, characterised in that a flow rate measuring device (8) and at least one laminar resistance (4) and at least one differential pressure controller (7) for maintaining the pressure drop at the laminar resistance (4) constant are arranged in the gas conduit (1) and that the apparatus is provided with a device to compensate for the effect of temperature.

12. Apparatus for carrying out the method as claimed in Claim 2 using a gas conduit leading to the gas consuming device, characterised in that at least one laminar resistance (4) and at least one turbulent resistance (9) are arranged in the gas conduit (1), and that at least one of these flow resistance is connected to a differential pressure measuring device (6) that the other flow resistances are connecte · a differential pressure controller (7) and that the apparatus is provided with a device to compensate for the effect of temperature.

13. Apparatus for carryling out the method as claimed in Claim 2 using a gas conduit leading to the gas consuming device, characterised in that at least one laminar resistance (4) and at least one turbulent resistance (9) are arranged in the gas conduit (1), and that all the flow resistances are connected in common to a differential pressure controller (7), that least one flow resistance is connected to a differential pressure measuring device (6) an that the apparatus is provided with a device to compensate for the effect of temperature.

14. Apparatus as claimed in Claim 12 or 13, characterised in that a flow rate measuring or metering device (8 or 3) is additionally arranged in the combustion gas conduit (1).

15. Apparatus for carrying out the method as claimed in Claim 3 using a gas conduit leading to the gas consuming device, characterised in that a laminar resistance (4a) which is connected to a differential pressure controller (7) and a flow rate transmission device (14) are arranged in the gas conduit (1), and that the flow rate transmission device (14) is additionally connected to a reference gas conduit (15) in which a laminar resistance (4b) which is connected to a differential pressure measuring device (6) is situated.

16. Apparatus for carrying out the method as claimed in Claim 4 using a gas conduit leading to the gas consuming device, characterised in that at least two laminar resistances (4a, 4b) and a turbulent resistance (9) are arranged in series in the gas conduit (1), and that a flow rate metering device (3) is connected to at least one laminat resistance (4a) and a differential pressure controller (7), and that a further laminar resistance (4b) and the turbulent resistance (9) are connected in common to a differential pressure measuring device (6) and that the apparatus is provided with a device to compensate for the effect of temperature.

17. Apparatus for carrying out the method as claimed in Claim 5 using a gas conduit leading to the gas consuming device, characterised in that a pressure reducer (2) is arranged in the gas conduit (1), and that the combustion gas conduit (1) is constructed thereafter in the form of two parallel branch flow conduits, that in each of the branch flow conduits at least one laminar resistance (4a, 4b) and a turbulent resistance (9a, 9b) are arranged in series, and that in the one branch flow conduit the laminar resistance (all the laminar resistances) is (are) arranged in front of the turbulent resistance and in the other branch flow conduit the laminar resistance (or the laminar resistances) is (are) arranged behind the turbulent resistance, and that a differential pressure controller (7) is connected to the combustion gas conduit (1) in front of and behind the splitting into the branch current conduits, that the two branch current conduits are connected together between the laminar and turbulent resistances by a bridge conduit (16) in which a differential pressure measuring device (6) and/or a flow rate measuring device (8) are arranged, and that the apparatus is provided with a device to compensate for the effect of temperature.

18. Apparatus as claimed in one of Claims 12 to 17, characterised in that the differential pressure measuring device (6) and the flow rate measuring device (8) are connected to a small computer (12).

19. A flow resistance as claimed in one of Claims 10 to 18, characterised in that the laminar resistance (4) is constructed as a tube with a circular or annular cross-section or as a corresponding bundle of tubes whose ratio of length to hydraulic diameter is at least 20 : 1, preferably 2000 : 1, and that each individual tube is rounded at its inlet and conically broadened at its end whereby the conical broadening has an angle to the tube axis of less than 20° preferably less than 10°.

20. A flow resistance as claimed in one of Claims 12 to 19, characterised in that the turbulent resistance (9) is constructed as an apertured partition whose ratio of hole diameter to length is greater than 2 : 1 and preferably is 5 : 1 to 20 : 1.

21. Apparatus as claimed in one of Claims 10 to 20, characterised in that the first laminar resistance (4) in the flow path is at least partially surrounded by a thermostat (5).

22. Apparatus as claimed in one of Claims 10 to 21, characterised in that the membrane of a differential pressure controller (7) is acted on in a known manner by a temperature and/or absolute pressure dependent force.

## Revendications

1. Procédé pour la mesure sans combustion et/ou la régulation de l'amenée de quantités de chaleur aux appareils d'utilisation du gaz, en particulier aux appareils d'utilisation du gaz naturel, caractérisé par le fait que la totalité du gaz combustible ou un courant partiel du gaz combustible passe à travers au moins une résistance d'écoulement qui cause un écoulement laminaire et une perte de charge et que l'on mesure le débit volumique de gaz et/ou la perte de charge dans au moins une résistance d'écoulement, les pertes de charge et/ou les débits volumiques non mesurés étant maintenus constants, que l'on compense l'influence de la température sur le système et que la (ou les) grandeur(s) mesurée(s) est (sont), conformément à la relation (4):

$$f(H) = a + b \cdot n^c \cdot \varrho^d \tag{4}$$

une mesure du pouvoir calorifique du gaz en écoulement, f (H) étant un pouvoir calorifique supérieur ou un pouvoir calorifique inférieur ou un indice de Wobbe, n la viscosité dynamique du gaz, $\varrho$ la masse volumique du gaz dans les conditions d'écoulement et a, b, c et d étant des constantes, et que l'on en tire un signal de mesure et/ou de régulation.

2. Procédé pour la mesure sans combustion et/ou la régulation de l'amenée de quantités de chaleur aux appareils d'utilisation du gaz, en particulier aux appareils d'utilisation du gaz naturel, caractérisé par le fait que la totalité du gaz combustible ou un courant partiel du gaz combustible passe à travers au moins deux résistances d'écoulement branchées à la suite l'une de l'autre et/ou branchées en parallèle, dont au moins une cause un écoulement laminaire et une autre, un écoulement turbulent, et des pertes de charge correspondantes et que l'on maintient constante la perte de charge dans au moins une résistance et qu'on la mesure sur l'autre (ou les autres) résistance(s) d'écoulement, ou que l'on maintient constante la perte de charge au travers de l'ensemble des résistances et qu'on la mesure sur au moins une résistance d'écoulement, que l'on compense l'influence de la température sur le système et que la (ou les) grandeur(s) mesurée(s) est (sont), conformément à la relation (4)

$$f(H) = a + b \cdot n^c \cdot \varrho^d \tag{4}$$

une mesure du pouvoir calorifique du gaz en écoulement, f (H) étant un pouvoir calorifique supérieur ou un pouvoir calorifique inférieur ou un indice de Wobbe, n la viscosité dynamique du gaz, $\varrho$ la masse volumique du gaz dans les conditions d'écoulement et a, b, c et d étant des constantes, et que l'on en tire un signal de mesure et/ou de régulation.

3. Procédé selon la revendication 1, caractérisé par le fait que, pour compenser l'influence de la température, on transmet la valeur mesurée du débit volumique de gaz ou de la perte de charge engendrée par une résistance d'écoulement à un appareil de mesure analogique qui est traversé par un autre gaz, de préférence de l'air.

4. Procédé selon la revendication 2, caractérisé par le fait que l'on conduit le gaz non seulement à travers les résistances d'écoulement laminaire et turbulent, mais encore à travers un appareil de mesure ou de dosage du débit volumique permettant de mesurer un débit de gaz ou de le maintenir constant.

5. Procédé selon l'une des revendications 2 et 4, caractérisé par le fait que l'on conduit le gaz ou un courar partiel du gaz à travers deux groupes disposés en parallèle, formés chacun de deux résistances d'écoulement branchées à la suite l'une de l'autre, dont une de chaque groupe cause un écoulement laminaire et la deuxième un écoulement turbulent de gaz, que dans l'un des groupes l'écoulement laminaire est assuré dans la première résistance d'écoulement et que, dans l'autre groupe, l'écoulement laminaire est assuré dans la dernière résistance d'écoulement, que l'on maintient constante la perte de charge au travers des deux groupes et que l'on mesure la perte de charge ou le débit volumique du gaz dans un pont disposé entre les deux groupes, entre les résistances d'écoulement laminaire et turbulent.

6. Procédé selon l'une des revendications 2, 4 et 5, caractérisé par le fait que les différences de pression et les débits volumiques mesurés ainsi qu'éventuellement − pour compenser leur influence sur le système − des valeurs mesurées de température et de pression absolue, sont transmis à un petit ordinateur qui les convertit en une impulsion de régulation ou de commande et/ou transmet des valeurs caloulées à un affichage et/ou à des appareils enregistreurs.

7. Procédé selon l'une des revendications 1 à 5, caractérisé par le fait qu'en plus de l'influence de la température, on compense aussi l'influence de la pression absolue sur le système.

8. Procédé selon l'une des revendications 1 à 5, caractérisé par le fait que, pur compenser l'influence de la température, on effectue au moins partiellement une régulation thermostatique de la première résistance laminaire dans le parcours d'écoulement.

9. Procédé selon la revendication 7, caractérisé par le fait, que pour compenser l'influence de la température et/ou de la pression absolue, on influence la membrane du régulateur de pression différentielle.

10. Dispositif pour la mise en oeuvre du procédé selon la revendication 1 avec utilisation d'une tuyauterie à gaz menant à l'appareil d'utilisation du gaz, caractérisé par le fait que dans la tuyauterie à gaz (1) sont disposés au moins un doseur de débit volumique (3) et au moins une résistance laminaire (4), que la résistance laminaire (4) est reliée à un appareil de mesure de pression différentielle (6) et que le dispositif est muni d'un appareil de compensation de l'influence de la température sur le système.

11. Dispositif pour la mise en oeuvre du procédé selon la revendication 1 avec utilisation d'une tuyauterie à gaz menant à l'appareil d'utilisation du gaz, caractérisé par le fait que dans la tuyauterie à gaz (1) sont disposés un appareil de mesure du débit volumique (8) et au moins une résistance laminaire (4) ainsi qu'au moins un régulateur de pression différentielle (7) servant à maintenir constante la perte de charge sur la résistance laminaire (4) et que le dispositif est muni d'un appareil de compensation de l'influence de la température.

12. Dispositif pour la mise en oeuvre du procédé selon la revendication 2 avec utilisation d'une tuyauterie à g\ ⋯enant à l'appareil d'utilisation du gaz, caractérisé par le fait que dans la tuyauterie à gaz (1) sont disposées au moins une résistance laminaire (4) et une résistance à turbulence (9) et qu'au moins une de ces résistance d'écoulement est reliée à un appareil de mesure de pression différentielle (6), que les autres résistances d'écoulement sont reliées à un régulateur de pression différentielle et que le dispositif est muni d'un appareil de compensation de l'influence de la température.

13. Dispositif pour la mise en ouevre du procédé selon la revendication 2 avec utilisation d'une tuyauterie à gaz menant à l'appareil d'utilisation du gaz, caractérisé par le fait que dans la tuyauterie à gaz (1) sont disposées au moins une résistance laminaire (4) et une résistance à turbulence (9) et que toutes les résistances d'écoulement sont reliées à un régulateur de pression différentielle commun (7), qu'au moins une résistance d'écoulement est reliée à un appareil de mesure de pression différentielle (6) et que le dispositif est muni d'un appareil de compensation de l'influence de la température.

14. Dispositif selon l'une des revendications 12 et 13, caractérisé par le fait qu'en outre, dans la tuyauterie à gaz (1) est disposé un appareil de mesure ou un doseur de débit volumique (8 ou 3).

15. Dispositif pour la mise en oeuvre du procédé selon la revendication 3, avec utilisation d'une tuyauterie à gaz menant à l'appareil d'utilisation du gaz, caractérisé par le fait que dans la tuyauterie à gaz (1) sont disposés une résistance laminaire (4a) qui est reliée à un régulateur de pression différentielle (7) ainsi qu'un appareil de transmission de débit volumique (14) et que l'appareil de transmission de débit volumique (14) est en outre relié à une tuyauterie de référence à gaz (15) dans laquelle se trouve une résistance laminaire (4b) qui est reliée à un appareil de mesure de pression différentielle (6).

16. Dispositif pour la mise en oeuvre du procédé selon la revendication 4, avec utilisation d'une tuyauterie à gaz menant à l'appareil d'utilisation du gaz, caractérisé par le fait que dans la tuyauterie à gaz (1) sont disposées à la suite l'une de l'autre au moins deux résistances laminaires (4a, 4b) et une résistance à turbulence (9) et qu'un doseur de débit volumique (3) est relié à au moins une résistance laminaire (4a) et à un régulateur de pression différentielle (7) et qu'une autre résistance laminaire (4b) et la résistance à turbulence (9) sont reliées à un appareil de mesure de pression différentielle (6) commun et que le dispositif est muni d'un appareil de compensation de l'influence de la température.

17. Dispositif pour la mise en oeuvre du procédé selon la revendication 5 avec utilisation d'une tuyauterie à gaz menant à l'appareil d'utilisation du gaz, caractérisé par le fait que dans la tuyauterie à gaz (1) est disposé un détendeur (2) et que la tuyauterie à gaz (1) est ensuite concue sous forme de deux tuyauteries parallèles à courant partiel, que dans chacune des tuyauteries à courant partiel sont disposées à la suite l'une de l'autre au moins une résistance laminaire (4a, 4b) et une résistance à turbulence (9a, 9b) et que dans l'une des tuyauteries à courant partiel, la résistance laminaire (ou les résistance laminaires) est (sont) disposée(s) avant la résistance à turbulence et que, dans l'autre tuyauterie à courant partiel, la résistance laminaire (ou les résistances laminaires) est (sont) disposée(s) après la résistance à turbulence et qu'un régulateur de pression différentielle (7) est relié à la tuyauterie à gaz (1) avant et après la division en tuyauteries à courant partiel, que les deux tuyauteries à courant partiel sont reliées entre elles entre les résistances laminaires et à turbulence par une tuyauterie de pont (16) dans laquelle sont disposés un appareil de mesure de pression différentielle (6) et/ou un appareil de mesure de débit volumique (8) et que le dispositif est muni d'un appareil de compensation de l'influence de la température.

18. Dispositif selon l'une des revendications 12 à 17, caractérisé par le fait que les appareils de mesure de pression différentielle (6) et l'appareil de mesure de débit volumique (8) sont reliés à un petit ordinateur (12).

19. Résistance d'écoulement selon l'une des revendications 10 à 18, caractérisée par le fait que la résistance laminaire (4) est conçue sous forme de tube à section circulaire ou annulaire ou de faisceau de tubes correspondant, dont le rapport de la longueur au diamètre hydraulique est d'au moins 20 : 1, de préférence compris entre 200 : 1 et 2000 : 1 et que chaque tube individuel est arrondi à l'entrée et élargi coniquement à l'extrémité, l'élargissement conique faisant avec l'axe du tube un angle plus petit que 20°, de préférence plus petit que 10°.

20. Résistance d'écoulement selon l'une des revendications 12 à 19, caractérisée par le fait que la résistance à turbulence, (9) est conçue sous forme de diaphragme à orifice dont le rapport du diamètre de l'orifice à la longueur est plus grand que 2 : 1 et, de préférence, compris entre 5 : 1 et 20 : 1.

21. Appareil selon l'une des revendications 10 à 20, caractérisé par le fait que la première résistance laminaire (4) dans le parcours d'écoulement est entourée au moins partiellement d'un thermostat (5).

22. Appareil selon l'une des revendications 10 à 21, caractérisé par le fait que la membrane d'un régulateur de pression différentielle (7) est soumise, de manière connue, à une force dépendant de la température et/ou de la pression absolue.

Fig.1

Fig.2

Fig.3

Fig.4

0 022 493

17

**Fig.5**

**Fig.6**

**Fig.7**